Europäisches Patentamt

(19) European Patent Office

Office européen des brevets

(11) Publication number : **0 424 158 B1**

(12) **EUROPEAN PATENT SPECIFICATION**

(45) Date of publication of patent specification :
30.03.94 Bulletin 94/13

(51) Int. Cl.⁵ : **A61K 7/06**

(21) Application number : **90311462.7**

(22) Date of filing : **18.10.90**

(54) Hair treatment composition.

(30) Priority : **20.10.89 GB 8923667**

(43) Date of publication of application :
**24.04.91 Bulletin 91/17**

(45) Publication of the grant of the patent :
**30.03.94 Bulletin 94/13**

(84) Designated Contracting States :
**AT BE CH DE DK ES FR GB GR IT LI NL SE**

(56) References cited :
**EP-A- 0 232 982**
**EP-A- 0 261 812**
**EP-A- 0 273 202**
**EP-A- 0 347 198**
**EP-A- 0 403 303**

(73) Proprietor : **UNILEVER PLC**
**Unilever House Blackfriars**
**London EC4P 4BQ (GB)**
(84) **GB**
Proprietor : **UNILEVER N.V.**
**Weena 455**
**NL-3013 AL Rotterdam (NL)**
(84) **BE CH DE DK ES FR GR IT LI NL SE AT**

(72) Inventor : **Gallagher, Peter**
**1 Mission Cottage, (Moss Lane)**
**Burscough, Lancashire (GB)**
Inventor : **Bowser, Paul Anthony**
**Dorset House, Latchford Road, Gayton**
**Wirral, Merseyside L60 3RW (GB)**
Inventor : **Marti, Vernon Peter John**
**44 Donne Avenue, Spital**
**Wirral, Merseyside L63 9YH (GB)**

(74) Representative : **Tonge, Robert James et al**
**UNILEVER PLC Patent Division Colworth**
**House Sharnbrook**
**Bedford MK44 1LQ (GB)**

EP 0 424 158 B1

Jouve, 18, rue Saint-Denis, 75001 PARIS

## Description

### FIELD OF INVENTION

The present invention relates to a hair treatment composition containing a low molecular weight compound which is intended to be retained in the hair shaft.

### BACKGROUND OF THE INVENTION

Certain 2-hydroxyalkanoic acids have been included in compositions for topical application to the skin, for the purpose of increasing extensibility and improving the appearance of the skin.

In EP 0 403 303 there is disclosed the combination of 2-hydroxyoctanoic acid, a conditioning agent and perfumes and colours. The function of the 2-hydroxyoctanoic acid is to increase elasticity of hair. There is no disclosure of the problem of enhancing delivery of perfume or dye to the hair shaft.

In GB 2 116 036 (Unilever) compositions are disclosed which comprise substituted octanoic acid (including 2-hydroxyoctanoic acid and 2-ketooctanoic acid) together with $C_{2-4}$ alkyl lactate. It is stated that these compositions improve the condition of the skin.

It is known that certain compounds enhance the penetration of dyes into the hair shaft. For example, EP 161 073 (Repligen) discloses the use of a range of compounds which are alcohols, phenols or esters, to aid the penetration of dopa and its analogues into the hair. Dopa is a dye precursor which is practically insoluble in water and solvents, and would penetrate the hair only slightly if applied on its own.

We have now found that certain 2-hydroxyalkanoic acids aid the penetration of some low molecular weight molecules into the hair shaft.

The low molecular weight compound is retained in the hair shaft more effectively than if 2-hydroxyalkanoic acids were not present in the composition.

### BRIEF SUMMARY OF THE INVENTION

Accordingly, the invention provides an aqueous hair treatment composition which comprises:
(i) from 0.05 to 5% by weight of a non-acidic compound being a hair dye or dye precursor having a molecular weight not over 5000; and
(ii) from 0.05 to 30% by weight of an acidic compound chosen from 2-hydroxyhexanoic acid, 2-hydroxyoctanoic acid, 2-hydroxydecanoic acid or mixtures thereof, to serve as hair shaft penetration enhancer for the non-acidic compound.

The invention further comprises a method for delivering a non-acidic low molecular weight compound to hair so as to penetrate into the hair shaft, which method comprises the step of contacting hair with an aqueous composition comprising:
(i) from 0.05 to 5% by weight of a non-acidic compound being a hair dye or dye precursor having a molecular weight not over 5000; and
(ii) from 0.05 to 30% by weight of an acidic compound chosen from 2-hydroxyhexanoic acid, 2-hydroxyoctanoic acid, 2-hydroxydecanoic acid or mixtures thereof, to serve as a hair shaft penetration enhancer for the non-acidic compound.

The invention still further comprises the use of an acidic compound chosen from 2-hydroxyhexanoic acid, 2-hydroxyoctanoic acid, 2-hydroxydecanoic acid, or mixtures thereof, as a hair shaft penetration enhancer for a nonacidic compound of molecular weight less than 5000, the non-acidic compound being chosen from hair dyes, dye precursors and perfumes.

### DETAILED DESCRIPTION OF THE INVENTION

#### The 2-hydroxyalkanoic acid

The composition of the invention comprises 2-hydroxyalkanoic acid chosen from 2-hydroxyhexanoic acid, 2-hydroxyoctanoic acid, 2-hydroxydecanoic acid or mixtures thereof in an amount from 0.05 to 30% by weight. The 2-hydroxyalkanoic acid is preferably 2-hydroxyoctanoic acid and is preferably present in an amount of from 0.5 to 5% by weight.

It is preferred that the pH of the hair treatment composition of the invention is maintained at below 7, most preferably at a pH of from 2.5 to 5. This may be achieved simply by the acidic nature of the hydroxyalkanoic

2

acid itself or preferably by the addition of a co-acid. The co-acid may be included in an amount sufficient to adjust the pH of the composition to a value of 2.5 to 5. A suitable amount is up to 5% by weight, preferably from 0.5 to 2% by weight.

The hair is an effective buffer and may cause the penetration enhancement effect of the 2-hydroxyalkanoic acid to be lost if the pH value of the composition is greater than 7.

Preferred examples of co-acids are citric acid and lactic acid.

The non acidic low molecular weight compound

The composition of the invention also comprises a non-acidic low molecular weight compound in an amount from 0.05 to 5% by weight.

The molecular weight is preferably not over 5000, most preferably below 3000. In general, compounds having a molecular weight of greater than 5000 are too large to penetrate the hair shaft in the relatively short contact times used for hair products.

The non-acidic low molecular weight compound may be, for example, a dye or dye precursor, which is preferably chosen from oxidative hair dyes, auto-oxidative hair dyes, metal chelatic dyes, direct dyes, melanin precursors or mixtures thereof.

Particularly suitable examples of hair dyes include

Food red 9 (CI No. 16185) sold under the trade name AMARANTH,

Food red 14 (CI No. 45430) sold under the trade name ERYTHROSINE,

Food red 3 (CI No. 14720) sold under the trade name CARMOSINE,

Food red 7 (CI No. 16255) sold under the trade name PONCEAU 4R,

Food yellow 13 (CI NO. 47005) sold under the trade name QUINOLINE YELLOW.

Acid Black (CI No. 28440) sold under the trade name BLACK PN.

When the non-acidic low molecular weight compound is a dye or a dye precursor, it is preferred that it is present in the composition of the invention in an amount of from 0.1 to 5% by weight.

The non-acidic low molecular weight compound for use in the composition of the invention may alternatively be a perfume.

Suitable examples of perfumes include eugenol, 2,6-dimethyl-7-octen-2-ol, ortho-tertiarybutyl-cyclohexyl acetate and 9-decen-1-ol, or proprietary perfumes containing mixtures of perfume components.

When the non-acidic low molecular weight compound is a perfume, it is preferably present in the composition in an amount of from 0.05 to 1% by weight.

The composition of the invention may also comprise certain ingredients known in the art and necessary to the particular formulation required. The composition may be formulated as a shampoo, conditioner, styling mousse or lotion, hair spray or setting composition or as any other composition suitable for application to the hair. Examples of other ingredients are surfactants, viscosity control agents, solubility control agents, foam boosters, opacifiers, perfumes, colouring agents, conditioning agents, preservatives, proteins, polymers, buffering agents and water.

PROCESS

The hair treatment composition of the invention is formulated by mixing together the required ingredients in the amounts specified.

ADVANTAGES OF THE INVENTION

In the hair treatment composition of the invention the 2-hydroxyalkanoic acid has been demonstrated to enhance the penetration of non-acidic low molecular weight compounds into the hair shaft. The composition therefore provides an improved delivery system for molecules such as hair dyes or perfumes. If a hair dye molecule, for example, penetrates the hair shaft, the colour is retained during washing and the original colour of the hair is seen only in the new hair growth. Similarly, if perfume molecules are delivered to the interior of the hair shaft in the presence of 2-hydroxyalkanoic acid, the perfume is discernable in the hair for much longer periods than if the perfume molecules were merely retained on the surface of the hair shaft.

COMPARATIVE TESTS

The effect of 2-hydroxyalkanoic acids on the penetration of non-acidic low molecular weight compounds is illustrated by the following Comparative tests.

Comparative test 1

Blonde hair switches were treated with a solution at pH 3.0 containing 2% by weight of 2-hydroxyoctanoic acid and 1% by weight of BLACK PN, a water-soluble acid black food dye. The switches maintained a dark grey colour which remained fast to washing for up to five shampoos. Control switches, treated with a similar solution containing no 2-hydroxyoctanoic acid, showed no darkening.

Comparative test 2

Hair switches were treated with a solution at pH 3.0 containing 1% by weight of 2-hydroxyoctanoic acid and 1% by weight of each of the water soluble anionic food dyes known as Ponceau 4R and Amaranth. Control hair switches were treated with a dye solution containing no 2-hydroxyoctanoic acid. It was shown that there is a significant increase in dyeing in the presence of 2-hydroxyoctanoic acid, and also that the anisotropy in dyeing is greatly reduced in the presence of the 2-hydroxyoctanoic acid.

Comparative test 3

Hair switches were treated with a solution at pH 3.0 containing 1% by weight of 2-hydroxyoctanoic acid, 1% by weight of lactic acid and 1% by weight of Ponceau 4R. Control hair switches were treated with a similar solution containing no lactic acid. The results indicated that dyeing enhancement by the 2-hydroxyoctanoic acid was markedly improved in the presence of lactic acid.

Comparative test 4

8cm/1.5g hair switches were placed for 5 minutes in 90g of a solution containing sodium lauryl ether sulphate with average two ethylene oxide residues (SLES 2EO), Eugenol and 1% by weight 2-hydroxyoctanoic acid (a hair: liquor ratio of 1:20 by weight) at pH 3.0. After removal from the solution, switches were rinsed under running water for 30 seconds and then dried at 55°C for 30 minutes. The switches were assessed by means of a paired-comparison test against control switches treated in a similar way but with a solution containing no 2-hydroxyoctanoic acid. Twelve panellists were asked to assess 6 pairs of switches, comparing strength and intensity of perfume over a period of 48 hours. The results of the assessment are set out in Table 1 below.

## Table 1, Perfume strength assessment

| System | Perceived perfume strength | | | | |
|---|---|---|---|---|---|
| | 0hrs | 1hr | 4hrs | 24hrs | 48hrs |
| 3% SLES 2EO 0.5% Eugenol | – | 0 | + | + | + |
| 3% SLES 2EO 0.05% Eugenol | 0 | 0 | 0 | 0 | 0 |
| 1.2% SLES 2EO 0.05% Eugenol | + | + | + | + | + |
| 0.3% SLES 2EO 0.05% Eugenol | 0 | + | + | + | + |

Notes

+    hair treated with treatment solution containing 1% by weight 2-hydroxyoctanoic acid perceived as having significantly stronger scent.

0    no significant difference perceived.

–    hair treated with control solution containing no 2-hydroxyoctanoic acid perceived as having significantly stronger scent.

All percentages are by weight.
The invention is further illustrated by the following Examples.

Example 1

The following is an example of a shampoo according to the invention.

| | % wt |
|---|---|
| Alpha-olefin sulphonate | 9.0 |
| Glucamate DOE 120 [1] | 4.5 |
| 2-Hydroxyoctanoic acid | 1.0 |
| Citric acid | 2.0 |
| Ortho t-butyl cyclohexyl acetate | 0.5 |
| Preservative, colour | qs |
| Water | to 100 |

1 - Glucamate DOE 120 is polyethylene glycol-120 methyl glucose dioleate.

The pH was adjusted to between 3 and 5 with triethanolamine.

Example 2

The following is an example of a shampoo according to the invention.

| | % wt |
|---|---|
| Alkyl benzene sulphonate | 9.0 |
| Glucamate DOE 120 | 4.5 |
| Ponceau 4R | 1.0 |
| 2-Hydroxyoctanoic acid | 0.5 |
| Perfume, preservative | qs |
| Water | to 100 |

The pH was adjusted to between 3 and 5 with triethanolamine.

Example 3

The following is an example of a shampoo according to the invention.

|  | % wt |
|---|---|
| Alkyl benzene sulphonate | 9.0 |
| Glucamate DOE 120 | 4.5 |
| 2-Hydroxydecanoic acid | 1.0 |
| Lactic acid | 3.0 |
| Carmosine | 1.0 |
| Perfume, preservative | qs |
| Water | to 100 |

The pH was adjusted to between 3 and 5 with triethanolamine.

Example 4

The following is an example of a conditioner according to the invention.

|  | % wt |
|---|---|
| Cetyl trimethyl ammonium chloride | 0.7 |
| Ceto/stearyl alcohol | 2.0 |
| Paraffin wax | 1.0 |
| Glycerol monostearate | 0.7 |
| 2-Hydroxyoctanoic acid | 1.0 |
| 2,6-Dimethyl-7-octen-2-ol | 0.4 |
| Preservative, colour | qs |
| Water | to 100 |

The pH was adjusted to between 3 and 5 with triethanolamine.

Example 5

The following is an example of a conditioner according to the invention.

|                                    | % wt    |
|------------------------------------|---------|
| Cetyl trimethyl ammonium chloride  | 0.7     |
| Ceto/stearyl alcohol               | 1.0     |
| Natrosol 250 HR $^2$               | 1.3     |
| Amaranth                           | 1.0     |
| 2-Hydroxyoctanoic acid             | 2.0     |
| Perfume, preservative              | qs      |
| Water                              | to 100  |

2 - Natrosol 250 HR is hydroxyethyl cellulose.

The pH was adjusted to between 3 and 5 with triethanolamine.

## Claims

1. An aqueous hair treatment composition which comprises:
   (i) from 0.05 to 5% by weight of a non-acidic compound being a hair dye or dye precursor having a molecular weight not over 5000; and
   (ii) from 0.05 to 30% by weight of an acidic compound chosen from 2-hydroxyhexanoic acid, 2-hydroxyoctanoic acid, 2-hydroxydecanoic acid or mixtures thereof, serving as a hair shaft penetration enhancer for the non-acidic compound.

2. An aqueous hair treatment composition as claimed in claim 1 wherein the acidic compound is 2-hydroxyoctanoic acid.

3. An aqueous hair treatment composition as claimed in claim 1 or claim 2 wherein the acidic compound is present in an amount from 0.5 to 5% by weight.

4. An aqueous hair treatment composition as claimed in any one of claims 1 to 3, wherein the composition further comprises a co-acid in an amount from 0.5 to 5% by weight.

5. An aqueous hair treatment composition as claimed in claim 4, wherein the co-acid is lactic acid or citric acid.

6. An aqueous hair treatment composition as claimed in any preceding claim, wherein the hair dye or dye precursor is chosen from oxidative hair dyes, auto-oxidative hair dyes, metal chelatic dyes, direct dyes, melanin precursors or mixtures thereof.

7. An aqueous hair treatment composition as claimed in any preceding claim wherein the composition is formulated as a shampoo.

8. An aqueous hair treatment composition as claimed in any one of claims 1 to 6 wherein the composition is formulated as a hair conditioner.

9. A method for delivering a non-acidic compound, being a hair dye or dye precursor having a molecular weight not over 5000, to hair so as to penetrate into the hair shaft, which method comprises the step of contacting hair with an aqueous composition comprising:
   (i) from 0.05 to 5% by weight of the non-acidic compound having a molecular weight not over 5000; and
   (ii) from 0.05 to 30% by weight of an acidic compound, chosen from 2-hydroxyhexanoic acid, 2-hydroxyoctanoic acid, 2-hydroxydecanoic acid or mixtures thereof, as a hair shaft penetration enhancer.

10. Use of an acidic compound chosen from 2-hydroxyhexanoic acid, 2-hydroxyoctanoic acid, 2-hydroxydecanoic acid, or mixtures thereof, as a hair shaft penetration enhancer for a non-acidic compound of molecular weight not over 5000, the non-acidic compound being chosen from hair dyes, dye precursors and perfumes.

11. Use of an acidic compound as claimed in claim 10, wherein the acidic compound is 2-hydroxyoctanoic acid.

12. Use of an acidic compound as claimed in claim 10 or 11 wherein the acidic compound is present in an amount from 0.5 to 5% by weight.

13. Use of an acidic compound as claimed in any one of claims 10 to 12, wherein the penetration is further enhanced by use of a co-acid in an amount from 0.5 to 5% by weight.

14. Use of an acidic compound as claimed in claim 13, wherein the co-acid is lactic acid or citric acid.

15. Use of an acidic compound as claimed in any preceding claim, wherein the said non-acidic compound is a hair dye or dye precursor.

16. Use of an acidic compound as claimed in claim 15, wherein the hair dye or dye precursor is chosen from oxidative hair dyes, auto-oxidative hair dyes, metal chelatic dyes, direct dyes, melanin precursors or mixtures thereof.


**Patentansprüche**

1. Wäßrige Haarbehandlungszusammensetzung, umfassend:
   (i) 0,05 bis 5 Gew.% einer nichtsauren Verbindung, bei der es sich um einen Haarfarbstoff oder -farbstoffvorläufer eines Molekulargewichts nicht über 5.000 handelt, und
   (ii) 0,05 bis 30 Gew.% einer sauren Verbindung, ausgewählt aus 2-Hydroxyhexansäure, 2-Hydroxyoctansäure, 2-Hydroxydecansäure oder Mischungen davon, die als Verstärker für ein Eindringen der nichtsauren Verbindung in den Haarschaft dient.

2. Wäßrige Haarbehandlungszusammenstzung nach Anspruch 1, wobei die saure Verbindung aus 2-Hydroxyoctansäure besteht.

3. Wäßrige Haarbehandlungszusammensetzung nach Anpruch 1 oder Anspruch 2, wobei die saure Verbindung in einer Menge von 0,5 bis 5 Gew.% vorliegt.

4. Wäßrige Haarbehandlungszusammensetzung nach einem der Ansprüche 1 bis 3, wobei die Zusammensetzung des weiteren eine Cosäure in einer Menge von 0,5 bis 5 Gew.% umfaßt.

5. Wäßrige Haarbehandlungszusammensetzung nach Anspruch 4, wobei die Cosäure aus Milchsäure oder Zitronensäure besteht.

6. Wäßrige Haarbehandlungszusammensetzung nach einem der vorhergehenden Ansprüche, wobei der Haarfarbstoff oder -farbstoffvorläufer aus oxidativen Haarfarbstoffen, autooxidativen Haarfarbstoffen, Metallchelatfarbstoffen, Direktfarbstoffen, Melaninvorläufern oder Mischungen davon ausgewählt ist.

7. Wäßrige Haarbehandlungszusammensetzung nach einem der vorhergehenden Ansprüche, wobei die Zusammensetzung als Shampoo formuliert ist.

8. Wäßrige Haarbehandlungszusammensetzung nach einem der Ansprüche 1 bis 6, wobei die Zusammensetzung als Haarkonditioner formuliert ist.

9. Verfahren zur Abgabe einer nichtsauren Verbindung, bei der es sich um einen Haarfarbstoff oder einen -farbstoffvorläufer eines Molekulargewichts nicht über 5.000 handelt, an das Haar, um in den Haarschaft einzudringen, umfassend den Schritt eines Inberührungbringens des Haars mit einer wäßrigen Zusammensetzung, die
(i) 0,05 bis 5 Gew.% der nichtsauren Verbindung eines Molekulargewichts von nicht über 5.000 und
(ii) 0,05 bis 30 Gew.% einer sauren Verbindung, ausgewählt aus 2-Hydroxyhexansäure, 2-Hydroxyoctansäure, 2-Hydroxydecansure oder Mischungen davon, als einen Verstärker eines Eindringens in den Haarschaft umfaßt.

10. Verwendung einer sauren Verbindung, ausgewählt aus 2-Hydroxyhexansäure, 2-Hydroxyoctansäure, 2-Hydroxydecansäure oder Mischungen davon, als Verstärker des Eindringens einer nichtsauren Verbindung eines Molekulargewichtes nicht über 5.000, ausgewählt aus Haarfarbstoffen, -farbstoffvorläufern und Duftstoffen, in den Haarschaft.

11. Verwendung einer sauren Verbindung nach Anspruch 10, wobei die saure Verbindung aus 2-Hydroxyoctansäure besteht.

12. Verwendung einer sauren Verbindung nach Anspruch 10 oder 11, wobei die saure Verbindung in einer Menge von 0,5 bis 5 Gew.% vorliegt.

13. Verwendung einer sauren Verbindung nach einem der Ansprüche 10 bis 12, wobei das Eindringen des weiteren durch die Verwendung einer Cosäure in einer Menge von 0,5 bis 5 Gew.% verstärkt wird.

14. Verwendung einer sauren Verbindung nach Anspruch 13, wobei die Cosäure aus Milchsäure oder Zitronensäure besteht.

15. Verwendung einer sauren Verbindung nach einem der vorhergehenden Ansprüche, wobei die nichtsaure Verbindung aus einem Haarfarbstoff oder -farbstoffvorläufer besteht.

16. Verwendung einer sauren Verbindung nach Anspruch 15, wobei der Haarfarbstoff oder -farbstoffvorläufer aus oxidativen Haarfarbstoffen, autooxidativen Haarfarbstoffen, Metallchelatfarbstoffen, Direktfarbstoffen, Melaninvorläufern oder Mischungen davon ausgewählt ist.

## Revendications

1. Composition aqueuse de traitement de cheveux, qui comprend :
(i) de 0,05 à 5% en poids d'un composé non acide qui est une teinture capillaire ou un précurseur de teinture dont la masse moléculaire ne dépasse pas 5000 ; et
(ii) de 0,05 à 30% en poids d'un composé acide choisi parmi l'acide 2-hydroxyhexanoïque, l'acide 2-hydroxyoctanoïque, l'acide 2-hydroxydecanoïque et des mélanges de ceux-ci, pour jouer le rôle d'un agent de rehaussement de la pénétration dans les tiges des cheveux du composé non acide.

2. Composition aqueuse de traitement de cheveux selon la revendication 1, dans laquelle le composé acide est l'acide 2-hydroxyoctanoïque.

3. Composition aqueuse de traitement de cheveux selon la revendication 1 ou 2, dans laquelle le composé acide est présent à raison de 0,5 à 5% en poids.

4. Composition aqueuse de traitement de cheveux selon l'une quelconque des revendications 1 à 3, dans laquelle la composition comprend en outre un co-acide à raison de 0,5 à 5% en poids.

5. Composition aqueuse de traitement de cheveux selon la revendication 4, dans laquelle le co-acide est l'acide lactique ou l'acide citrique.

6. Composition aqueuse de traitement de cheveux selon l'une quelconque des revendications précédentes, dans laquelle la teinture capillaire ou le précurseur de teinture est choisi parmi les teintures capillaires oxydantes, les teintures capillaires auto-oxydantes, les teintures de métaux chélatiques, les colorants directs, les précurseurs de mélanine ou leurs mélanges.

7. Composition aqueuse de traitement de cheveux selon l'une quelconque des revendications précédentes, dans laquelle la composition est formulée en schampooing.

8. Composition aqueuse de traitement de cheveux selon l'une quelconque des revendications 1 à 6, composition qui est formulée en agent de conditionnement des cheveux.

9. Procédé de distribution d'un composé non acide qui est une teinture capillaire ou un précurseur de teinture dont la masse moléculaire ne dépasse pas 5000, aux cheveux de façon à pénétrer dans les tiges des cheveux, procédé qui consiste à mettre en contact les cheveux avec une composition aqueuse comprenant :
   (i) de 0,05 à 5% en poids d'un composé non acide qui est une teinture capillaire ou un précurseur de teinture dont la masse moléculaire ne dépasse pas 5000 ; et
   (ii) de 0,05 à 30% en poids d'un composé acide choisi-parmi l'acide 2-hydroxyhexanoïque, l'acide 2-hydroxyoctanoïque, l'acide 2-hydroxydecanoïque et des mélanges de ceux-ci, pour jouer le rôle d'un agent de rehaussement de la pénétration dans les tiges des cheveux du composé non acide.

10. Utilisation d'un composé acide choisi parmi l'acide 2-hydroxyhexanoique, l'acide 2-hydroxyoctanoïque, l'acide 2-hydroxydecanoïque ou des mélanges de ceux-ci, en qualité d'un agent de rehaussement de la pénétration dans les tiges des cheveux par un composé non acide dont la masse moléculaire ne dépasse pas 5000, le composé non acide étant une teinture capillaire, un précurseur de teinture ou un parfum.

11. Utilisation d'un composé acide selon la revendication 10, dans laquelle le composé acide est l'acide 2-hydroxyoctanoïque.

12. Utilisation d'un composé acide selon la revendication 10 ou 11, dans laquelle le composé acide est présent à raison de 0,5 à 5% en poids.

13. Utilisation d'un composé acide selon l'une quelconque des revendications 10 à 12, dans laquelle la pénétration est encore améliorée par l'emploi d'un co-acide en une proportion de 0,5 à 5% en poids.

14. Utilisation d'un composé acide selon la revendication 13, dans laquelle le co-acide est l'acide lactique ou l'acide citrique.

15. Utilisation d'un composé acide selon l'une quelconque des revendications précédentes, dans laquelle ledit composé non acide est une teinture capillaire ou un précurseur de teinture.

16. Utilisation d'un composé acide selon la revendication 15, dans laquelle la teinture capillaire ou le précurseur de teinture est choisi parmi les teintures capillaires oxydantes, les teintures capillaires auto-oxydantes, les teintures de métaux chélatiques, les colorants directs, les précurseurs de mélanine ou leurs mélanges.